# EUROPEAN PATENT SPECIFICATION

(11) **EP 4 149 581 B1**
(45) Date of publication and mention of the grant of the patent: **27.03.2024**
(21) Application number: 20816962.3
(22) Date of filing: 30.11.2020
(51) Int. Cl.: A61L 31/12, A61B 17/82, A61B 17/86, B29C 43/02, B29C 43/34, B29C 43/36, B29C 70/46, B30B 7/04, A61B 17/80, B29B 11/16, B29C 33/30, B29C 43/18, B29C 43/52, B29C 45/14, B29C 45/33, B29C 70/24, B29C 70/44, B29C 70/68, B29C 70/84, B29L 1/00, B29L 31/00

(54) **METHOD FOR MANUFACTURING FIBER REINFORCED ARTICLE AND APPARATUS**
VERFAHREN UND VORRICHTUNG ZUR HERSTELLUNG EINES FASERVERSTÄRKTEN ARTIKELS
PROCÉDÉ DE FABRICATION D'UN ARTICLE RENFORCÉ PAR DES FIBRES ET APPAREIL

(43) Date of publication of application: 22.03.2023
(73) Proprietor: Arctic Biomaterials Oy, 33520 Tampere (FI)
(72) Inventor: HUTTUNEN, Mikko, 33520 Tampere (FI); VUORISALO, Vesa, 33520 Tampere (FI); HEINO, Harri, 33520 Tampere (FI)
(74) Representative: Kolster Oy Ab
(86) International application number: PCT/EP2020/083887
(87) International publication number: WO 2022/111826

(56) References cited:
- EP-A1- 1 506 882
- EP-A2- 0 373 294
- US-A- 4 208 174
- US-A1- 2005 150 444
- US-A1- 2016 243 732
- US-A1- 2017 087 620
- US-A1- 2018 244 006
- US-B1- 8 043 553

## Description

### TECHNICAL FIELD

The present invention relates to an apparatus and a method of manufacturing a fiber reinforced article, and more particularly to a fiber reinforced article having a tailored multidirectional fiber orientation.

### BACKGROUND ART

Composite materials, also referred to as composites, are a combination of two or more materials that are mixed or joined on a macroscopic level. They are used in engineering applications where a pure material cannot provide the specific set of properties that are required. They can be thought of as a single material that has been enhanced by the addition of another material.

Fibers are added as a means of reinforcement and provide strength, stiffness, or any other desired property to the composite. One of the problems associated with the above arrangement is how to preserve the desired multidirectional fiber orientation of a complex-shaped article during the manufacturing phase.

US 2018/244006 A1 discloses a method and device for making double-toothed belt where the die segments are not in direct contact with each other during the initial position and movement. US 4208174 A discloses a radial compression molding apparatus where ethe die segments are in direct contact with each other during the initial position and movement and in the compressed position. EP 0373294 A2 discloses a method for producing FRP screw-like fastening elements where a heated rod is axially pressed to laterally expand rod material,

### SUMMARY

An object of the present invention is thus to provide a method to solve the above problems related to manufacturing the articles with desired fiber orientations. The desired fiber orientation may be just a simple uniaxial orientation or complex multilayer multidirectional orientation depending on the end product requirements. The objects of the invention are achieved by a method and an apparatus which are characterized by what is stated in the independent claims. The preferred embodiments of the invention are disclosed in the dependent claims.

The invention is based on the idea of using radial molding and forming a fiber reinforced article, which comprises a tailored fiber orientation, from the mold cavity, wherein the continuous reinforcing fibers follow to a surface design of said article.

### BRIEF DESCRIPTION OF THE DRAWINGS

In the following the invention will be described in greater detail by means of preferred embodiments with reference to the attached drawings, in which
Figure 1 is a flow chart of a method according to an embodiment;
Figures 2 and 3 illustrate exemplary die segments;
Figure 4 illustrates an exemplary composite preform;
Figures 5a-5f illustrate a series of exemplary manufacturing steps with pistons; and
Figure 6 illustrates an alternative step of the exemplary manufacturing step.

### DETAILED DESCRIPTION OF EMBODIMENTS

This invention is focused on fiber reinforced thermoplastic polymer composites, which can be especially used in medical devices such as bone screws and fasteners. Said medical devices are usually made of stainless steel or titanium alloys. However, these materials tend to have certain challenges, such as mechanical mismatch with mechanical properties of bone tissue which may cause further challenges such as stress shielding. Metallic implant may also cause disturbance in imaging such as in MRI. This has led engineers to search for alternative materials which can meet the desired effects and properties.

One of the alternatives is the fiber reinforced polymer composite. This type of composites is composed of a combination of reinforcing fibers, and a thermoplastic polymer matrix material, that surrounds the fibers. Fibers add strength and stiffness to an otherwise viscoelastic polymer that, without reinforcement, lacks the mechanical properties needed in certain applications. Fibers and matrix work together in synergy providing a composite material with characteristic properties benefiting from the contribution of both elements.

Fibers are added as a means of reinforcement and provide strength, stiffness, or any other desired property to the composite. The matrix bonds the fibers together, protects the fibers from damage, and distributes the load from one fiber to another. The properties of the composite are determined by the properties of the fibers, their length, diameter, orientation, and amount, as well as the properties of the matrix, and the bonding between the matrix and the fibers. When using reinforcing fibers, the fibers can be chopped and/or continuous.

When the continuous fibers are aligned, they provide maximum strength along the direction of alignment. The composite can be considerably weaker along other directions and can therefore be highly anisotropic. This anisotropy can be overcome by fibers aligned in desired directions, i.e. fiber orientations.

One of the problems associated with the above arrangement is how to preserve the desired fiber orientation of a complex-shaped article during the manufacturing phase. On laminated planar structures this can be easily overcome by aligning fibers on desired fiber orientations on distinct layers or by selecting premanufactured sheets with desired fiber orientations. If the fiber reinforced article being manufactured is a simple rotationally symmetrical object the desired fiber orientation can be achieved by means of filament winding. On more complex shapes the desired fiber orientation may be manufactured by means of automatic composite manufacturing techniques, such as automatic tape placement and/or automatic fiber placement. If, however, the complex fiber orientation is to be manufactured on small size products which need to be mass produced on large quantity on industrial scale, the preferable manufacturing techniques include compression molding and injection molding.

During the conventional compression moulding, the fiber orientation of a preform changes or it is considerably challenging to preserve on certain geometry articles. Preserving the desired fibre orientation on planar structures is somewhat straightforward, but more complex geometries are not that easy to reproduce with predictable fibre orientation. For example, when compression molding a rotationally symmetrical solid object, the shape transformation of a preform is not uniform along the surfaces of the object when compressing the preform to mold defining the final shape of the article. Thus, the initial fiber orientation of preform distort, shuffle and/or disarrange at different amounts on different sections due to non-uniform shape transformation.

On injection molding the desired fiber orientation of the article being manufactured can be achieved by using an insert containing the fibers having desired fiber orientation. The insert is placed inside the mold defining the final shape of the article before injecting the matrix polymer which fills the mold. The largest outer dimensions of the insert need to be smaller than the smallest outer dimensions of the mold, because otherwise the fiber insert would squeeze on between the mold parts when the mold closes making the injection molding impossible.

The present invention relates to a continuous fiber reinforced structure forming method, which utilizes radial molding. Continuous fiber reinforced structure obtained by this method for certain product geometries can comprise a layer structure with defined fiber orientation on each layer which can be tailored having desired properties. Using conventional methods such as compression molding, such layer structure and/or fiber orientation tends to shuffle and disarrange during the molding, which causes the desired properties of the finished product to weaken and on some occasion, even render it useless. Using radial molding, the construction/structure of desired fiber orientation of final product can be tailored/predicted as the shape transformation during the mold closing is uniform radially surrounding the longest dimension of the preform. Ideally the fiber orientation structure can even remain unchanged in the finished product, which maximizes benefits of the desired properties. Such properties can be, for example, toughness, stiffness, or any other desired mechanical property.

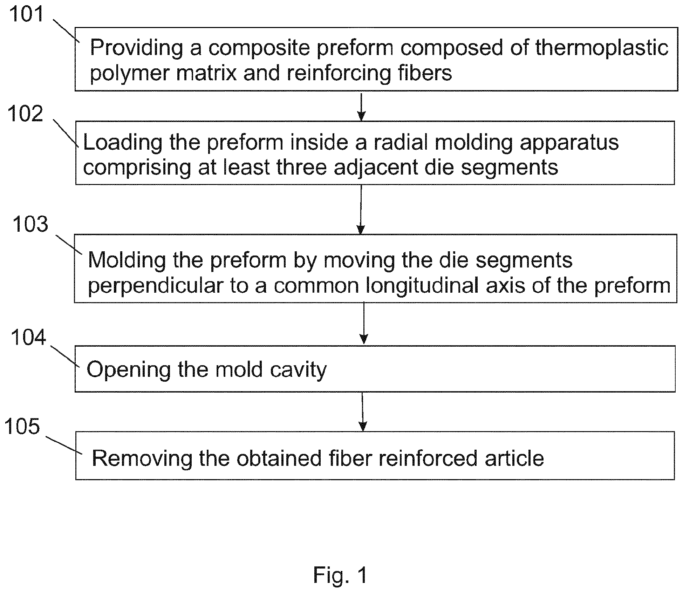

Figure 1 illustrates a flow chart of a method of forming a fiber reinforced article according to an embodiment. The first step 101 comprises providing a composite preform composed of thermoplastic polymer matrix and reinforcing fibers, wherein the preform comprises an initial volume and initial fiber orientation. The preform may be rotationally symmetrical and comprise an intermingled or interlocked layer structure, which would be preserved in the finished article. The preform may also comprise a tailored fiber structure, on which the fibers are initially located in separate and superimposed layers, and on which there are designed gaps on fiber structure on undermost layers and extra fibers on layers above the layers having gaps. These extra fibers on topmost layers may be located on the same position as the gaps on undermost layers. Thus, when radially compressing the preform, the extra fibers on topmost layers move to direction of the core and fill the gaps and the fiber structure of final product is 3D intermingled or interlocked structure, which is created during the radial molding phase. Each layer may consist of same or different material, and additionally may comprise a helix orientation having a desired angle such as 90°, 45° or 30°, for instance. The preform may consist of a core or insert wrapped around multiple layers having different helix, uniaxial, etc. orientation. In some structures, there may be gaps between different layers which would further facilitate interlocking the fibers in the finished article.

The thermoplastic materials are materials which shape can be transformed when heating them to certain material specific temperatures (e.g. glass transition temperature, melting temperature, etc.). Certain material properties, such as degree of crystallinity, strength properties, etc., of thermoplastic polymers may also be altered as a function of temperature and time. When thermoplastics are heated to certain temperature (glass transition temperature Tg), they soften, and their shape can be transformed. Semi-crystalline and crystalline thermoplastic polymers melt when they are heated to their melting point (Tm) or above melting point. Amorphous thermoplastic polymers do not have a melting point. Usually thermoplastic polymers are molded on temperatures above the Tg or above the Tm. They solidify to a glassy state when cooled below their glass transition temperature. There are many different types of fibers that can be used to reinforce polymer matrix composites. The most common are carbon fibers (AS4, IM7, etc.) and fiberglass (S-glass, E-glass, etc.). Fibre preforms are often manufactured in sheets, continuous mats, tubular structures or as continuous filaments or continuous filaments/tapes impregnated using matrix polymer.

The second step 102 comprises loading the preform inside a radial molding apparatus, either manually or automatically by a machine. Said radial molding apparatus comprises at least three adjacent die segments next to each other forming a mold cavity having an initial volume in an initial position and a final volume in a compressed position, which is smaller than the initial volume. The final volume of the mold cavity also defines a final volume and shape of the finished fiber reinforced article.

Each die segment can be identical, such as wedge-shaped with planar surfaces, which are arranged to form an approximately cylindrical central cavity. However, other shapes can also be formed depending on the design of the cavity surface. The wedges can be hinged and driven in unison to change the diameter, and consequently the volume, of the cavity. The die segment can have any design comprising two adjacent sides forming a 120° or less angle.

The third step 103 comprises molding the preform by moving said die segments. Each die segment is in direct contact with each other during end positions and movement of the mold, such as opening and closing as well as during the initial and compressed position. During this step, the compression applies similar or identical radial force and deformation towards the longitudinal axis of the preform.

Radial molding is a technique where the molded article is formed by a moldable continuous fiber reinforced preform material from the initial volume to the final volume along a plurality of radial directions. These radial directions are substantially perpendicular to a common longitudinal axis and arranged to lie in different planes. In this context the common longitudinal axis refers to the axis along which preform is loaded and perpendicular to the compression.

The said movable radial die segments are movable concurrently between the initial position and the final compression position which respectively define said initial volume and said final volume. The volume of the mold cavity in the compressed position can be smaller than or equal to the volume of the preform. Additionally, the volume of the preform can be smaller than the volume of the mold cavity in the initial position, and transverse dimension of the preform in the initial position can be larger than the transverse dimension of finished article in the compressed position. The transverse dimension of the preform in the initial position can also be smaller than or equal to the transverse dimension of the finished article in the compressed position, wherein the preform is additionally compressed from an end along the common longitudinal axis of the preform.

Linear or curved path actuators can be used to move said die segments between their initial and compressed positions. The said radial die segments can move along either linear or curved paths during which the interface surfaces on between the adjacent radial die segments can be then either linear or curved.

The radial mold comprised of at least three mold die segments described in this invention may also be used as a mold for injection molding. In injection molding the mold which opens radially, as described in this invention, enables to use continuous fiber inserts which have even larger initial diameter than the molded final product. With conventional mold there are always gaps on between the mold parts, also known as dies, when the mold opens and thus the use of such larger diameter inserts would be impossible, as the insert would get squeezed between the mold parts when the mold closes. If the mold opens and closes radially as explained in this invention such squeezing will not happen. This enables to use such continuous fiber reinforced inserts in overmolding injection molding which are impossible to use when using any other type of molds in injection molding.

The fourth step 104 comprises opening the mold cavity, by returning the die segments to the initial position, wherein each die segment is in direct contact with adjacent die segments during the movement. Both compression and release movements can be actuated by actuators to forcedly move said die segments between their initial and compressed positions. Said actuator can be hydraulic power cylinder, for instance, attached to the die segments. Operation of the die segments can be controlled by a valve. The power cylinders can be simultaneously connected to compress and, after compression, to reverse flow through the valve.

The fifth step 105 comprises removing the obtained fiber reinforced article from the mold cavity, either manually or automatically by a machine. The finished article comprises a tailored orientation and layer structure, wherein the continuous reinforced fibers follow or conform to a surface contour of said article. The initial fiber orientation structure can even remain unchanged in the finished article, which maximizes benefits of the desired properties. The obtained article comprises desired properties such as better toughness, as well as compression, torsion, impact resistance, or any other desired property.

In another embodiment, the method further comprises a heating step, wherein the preform is heated to above the glass transition temperature or melting temperature of matrix polymer. Heating the preform facilitates formability of the preform. Said heating step can be arranged inside the radial molding apparatus before the molding step or before loading the preform inside the radial molding apparatus. In some embodiments, the die segments can be heated using heating elements placed inside the desired locations in the compression die body and transfer the heat to the preform located in mold cavity. The preform is then cooled down inside the mold cavity by conduction or the finished article is cooled down after the molding step by any suitable cooling means such as air cooling.

Before or after or during the molding step, the method further comprises sliding at least one piston inside the mold cavity along the common longitudinal axis of the preform to further facilitate the compression of the preform by sealing the mold cavity from at least one end. In this context, the piston refers to any rod or stick or similar arranged to fit and move inside the molding apparatus. For example, one piston on each longitudinal end of the mold cavity can be provided, and during molding step, said pistons slide towards the preform and thus further compresses the preform from both ends. With the combination of the mold segments and the pistons all sides of the product can be formed. Preferably the pistons slide towards the preform after the die segments are moved to final compression position. A separate actuator may move the piston or both pistons. The piston may comprise a feature penetrating the whole mold cavity from one piston to another piston. Such embodiment is beneficial particularly when molding parts which have hollow opening trough the longest dimension of part. An example of such part is a cannulated screw.

A distal end of the piston may comprise a mold with an inverse design which is reproduced at an end portion of the obtained fiber reinforced article. The mold can be made of plastic such as polyetheretherketone (PEEK), which has excellent mechanical and chemical resistance properties that are maintained at high temperatures. The distal end in this context refers to the end contacting the preform. The design can be for instance a screw head and/or screw tip. On another embodiment the piston can include a separate part which is used as an insert and which is joined to the part being manufactured during the radial molding phase. Such insert is temporarily attached to piston prior to compression molding phase and it is permanently attached to the part being radial molded during the molding phase. Such insert may be composed of same material as part being manufactured or it may be composed of different material such as a metal, ceramic, etc. Such insert may comprise the whole tip of the part being manufactured. Such tip can be for instance threaded and used in applications where a mixture of two or more materials is more advantageous.

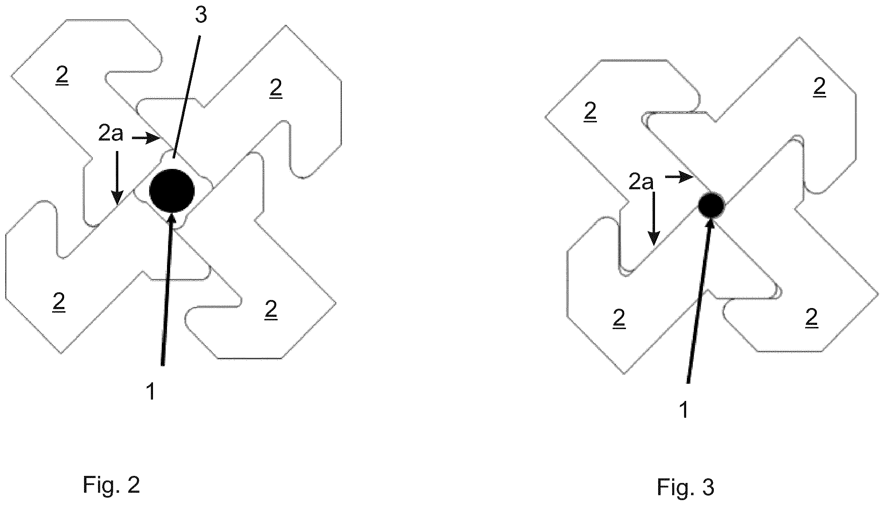

In yet another embodiment, before the molding step, the method further comprises sliding at least one piston with specifically designed mold sealing feature at the distal end of the piston along the common longitudinal axis of the preform to further facilitate mold sealing. The sealing feature stops at the edge of the mold cavity. In this context, the piston includes a geometrical feature which seals the mold at the stage where mold cavity is "open" (figure 2) before the die segments move to the compressed position (figure 3). On such embodiment the separate actuator may move the piston or both pistons.

Figures 2 and 3 illustrate exemplary die segments 2 of a radial molding apparatus viewed along a longitudinal axis in an initial position and a compressed position, respectively. As shown in Figs. 2 and 3, the apparatus comprises four die segments 2 forming a mold cavity 3. The die segments 2 can be similar or different depending on the manufactured product. Each die segment 2 comprises two interface surfaces 2a forming a wedge, which are in contact with interface surfaces 2a of adjacent die segments 2 all the time during an initial position (Fig. 2), molding step and compressed position (Fig. 3) and during movements between the positions. The die segments 2 can move along linear path which the interface surfaces 2a between the adjacent die segments 2 can be linear.

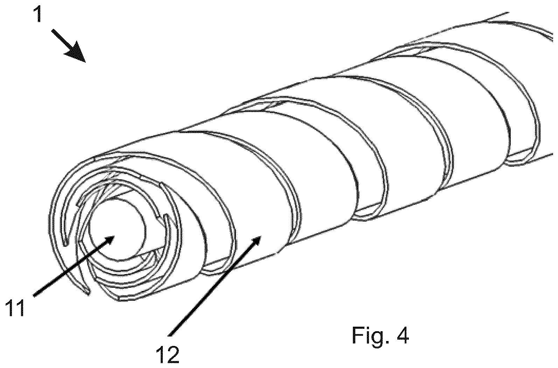

Figure 4 illustrates an exemplary composite preform 1 comprising thermoplastic polymer matrix and reinforcing fibers. The preform 1 comprises a core 11 having uniaxial fiber orientation and plurality surrounding fiber layers 12 having a substantially 45° helix orientation. The helix orientation may be either or both right hand or/and left hand. The layers 12 may be overlapped with 45°/-45°/45°/- 45°/45° etc. helix orientation, for example. The layers 12 may overlap with different or same helix orientation from the core 11 to the surface, wherein the fibers of the core 11 comprise same orientation parallel to the longitudinal axis. In some examples, the layers 12 may have same helix orientation close to the core 11 and different helix orientation close to the surface. The continuous reinforcing fibers are arranged to follow or conform to a surface design of finished article. In screws especially, the helix angle is essential for determining torque. Screw efficiency is controlled by the helix angle, and the maximum efficiency is between 40 and 45 degrees. However, in some embodiments, the helix orientation can be substantially 15° or 30° or parallel to the longitudinal axis. The fiber insert 11 may also be replaced with a different material.

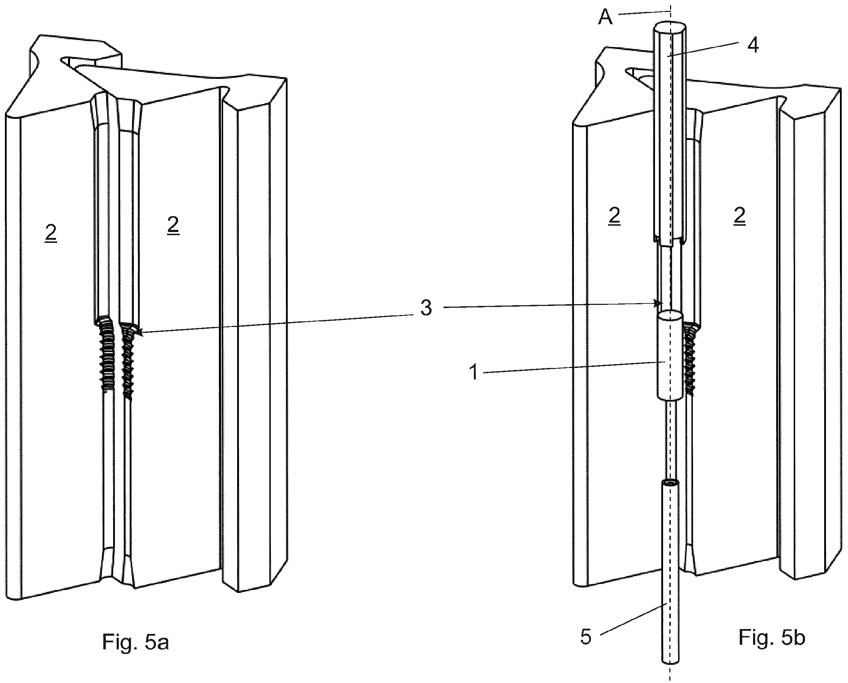

Figures 5a-5f illustrate a series of exemplary manufacturing steps comprising similar die segments from Figures 2 and 3 with pistons 4, 5 in open views, wherein two front adjacent die segments 2 are not shown. Figure 5a illustrates the die segments 2 in the initial position, wherein a mold cavity 3 has an initial volume. The mold cavity 3 in this example has a shape of a threaded screw. Above and below the mold cavity 3 are paths for pistons.

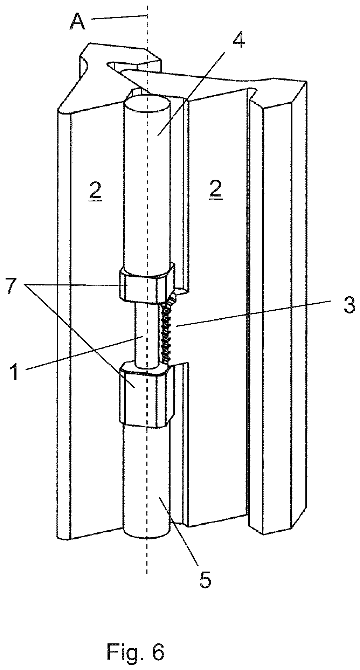

Figure 5b illustrates the die segments 2 and pistons 4, 5 in initial positions, wherein a preform 1, having an initial shape and volume and comprising thermoplastic polymer matrix and reinforcing fibers, has been loaded inside the mold cavity 3. The preform 1 has a common longitudinal axis A which is perpendicular to compression. The initial volume of the preform 1 can be smaller than the initial volume of the mold cavity 3, and in a compressed position the mold cavity 3 and a finished article has a matching volume. The first piston 4 and the second piston 5 may have different diameter and shape but they may also have same diameter (as shown in Fig. 6). The pistons 4, 5 can be made of metal or metal with plastic tip such as PEEK.

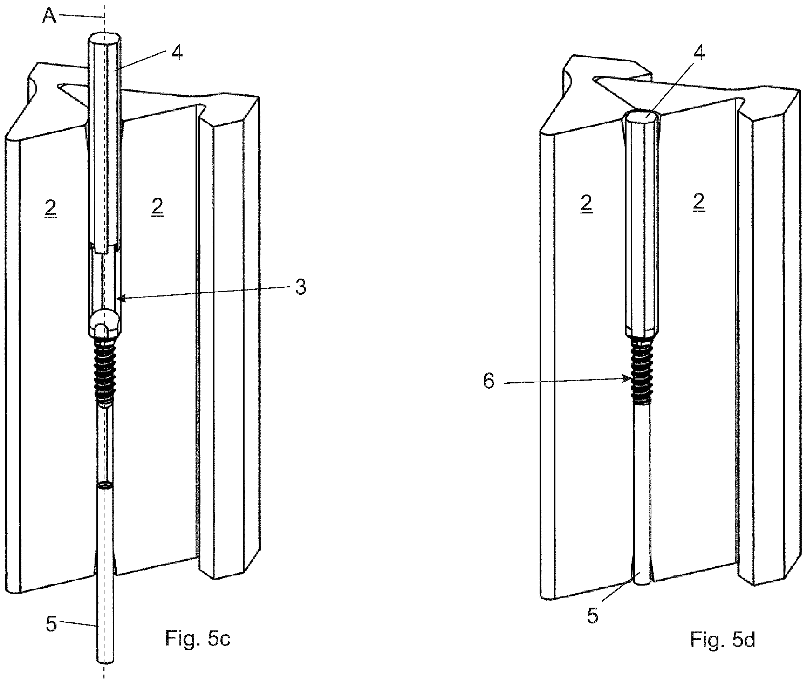

Figure 5c illustrates a step, wherein the die segments 2 are at the compressed position while the pistons 4, 5 are still at the initial position. During radial compression, longitudinal shape of the preform 1 is formed and having a tailored fiber orientation wherein the continuous reinforcing fibers follow or conform to the surface contour. As seen from Fig. 5c, both end sections have a protrusion with non-controlled shape (shown as round shape in Fig. 5c), which may not be a desired design.

Figure 5d illustrates the complete compressed position, wherein both pistons 4, 5 have slid inside the mold cavity 3 after the radial compression. The volume of the mold cavity 3 in the compressed position equals to the volume of the finished article 6. The first piston 4, and/or the second piston 5, has a distal end, which is the end contacting the preform 1 or unfinished article, that functions as a mold with an inverse design which is reproduced at an end portion of the finished article 6.

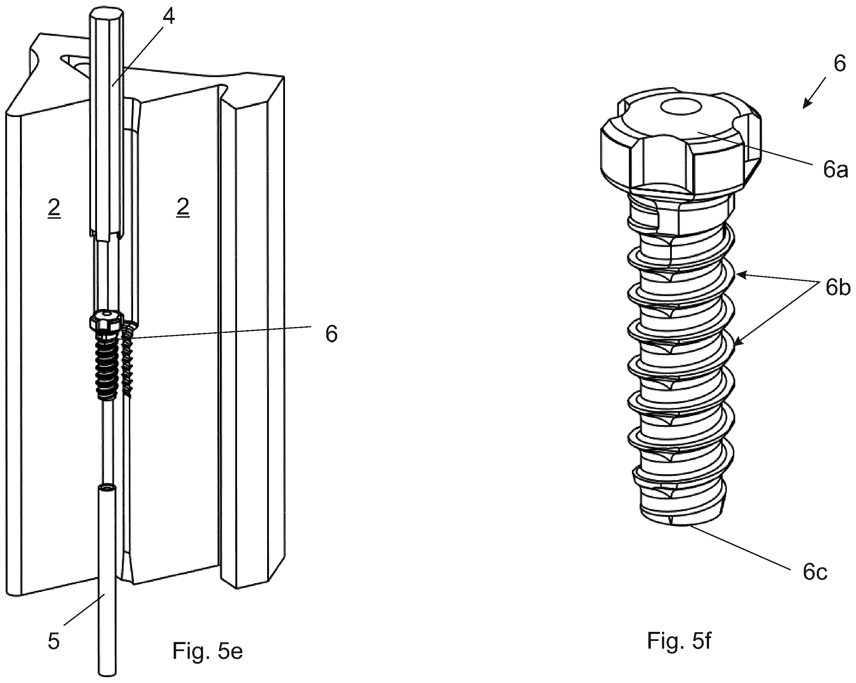

Figure 5e illustrates the step, where the die segments 2 and pistons 4 and 5 are returned to the initial position and the finished article 6 is ready to be removed.

Figure 5f illustrates a closer view of the finished article 6, which can be divided into the end portion 6a, middle portion 6b and tip portion 6c. The end portion 6a has a top shape which is formed by the first piston 4. The middle portion 6b has the helix threads which have the tailored fiber orientation with optimized mechanical properties. The tip portion 6c can be flat or, in some embodiments, include a tip made of other material than the preform 1. It may be fully threaded or partially threaded or smooth.

Figure 6 illustrates an alternative step of the exemplary manufacturing steps. The embodiment of Figure 6 is very similar to the one explained in connection with Figure 5c. Therefore, the embodiment of Figure 6 is in the following mainly explained by pointing out differences.

Figure 6 illustrates a step before the radial compression, wherein the pistons 4, 5 are at the compressed position while the die segments 2 are still at the initial position (i.e. 5b). The pistons 4, 5 can either compress the end portion of the preform 1 to the desired design before radial compression or keep the preform 1 from flowing out of the mold cavity 3 during the radial compression while the radial compression pressure causes the preform 1 to push towards the pistons 4, 5 or both. In both cases the finished product is obtained having a tailored fiber orientation wherein the continuous reinforcing fibers follow or conform to the surface contour.

In a yet another embodiment, the piston 4, 5 may further comprise a sealing feature 7 at the interface of the mold cavity 3 along the common longitudinal axis A of the preform 1 to further facilitate mold sealing. The term "interface of the mold cavity 3" in this context refers to the border where the mold cavity 3 is defined by the final volume. The sealing feature 7 is shaped to seal the interface of at proximal and/or distal end of the mold cavity 3 when the die segments 2 are still at the initial position and arranged to focus and lock against rotation of the die segments 2 at the compressed position. The sealed interface at the end of the mold cavity 3 thus prevents leakage of moldable preform 1 to these directions and thus the sealed interfaces improve pressure regulation during the molding phase. When the sealing feature 7 is utilized, the diameter of the pistons 4, 5 may be smaller than the diameter of the mold cavity 3 in the compressed stage. The sealing feature 7 may be manufactured of any suitable sealant material such as rubber, metal or plastics such as PEEK. The sealing feature 7 may also be composed of same material as the rest of the pistons. The sealing feature 7 may also be a geometrical feature of the piston 4, 5, which is integrated or seamlessly joined to the piston 4, 5 and which is composed of any suitable material.

## Claims

1. A method of forming a fiber reinforced article comprising following steps:
101) providing a composite preform (1) comprising of thermoplastic polymer matrix and reinforcing fibers, wherein the preform (1) comprises an initial volume and initial fiber orientation,
102) loading the preform inside a radial molding apparatus comprising of at least three adjacent die segments (2) next to each other forming a mold cavity (3) in an initial position having an initial volume,
103) molding the preform (1) by moving the die segments (2), which are in direct contact with each other during the initial position and movement and a compressed position, and which are perpendicular to a common longitudinal axis (A) of the preform (1), wherein the initial volume of the mold cavity (3) decreases and the die segments (2) compress the preform (1) to a form defined by the mold cavity (3) in the compressed position having a final volume, which is smaller or equal to the initial volume of the preform (1),
wherein before or after the molding step, at least one piston (4, 5) is slid towards the preform (1) inside the mold cavity (3) along the common longitudinal axis (A) of the preform (1) to further facilitate the compression of the preform (1) by sealing the mold cavity (3) from at least one end,
104) opening the mold cavity (3), and
105) removing the obtained fiber reinforced article (6), which comprises a tailored fiber orientation, from the mold cavity (3), wherein the continuous reinforcing fibers follow to a surface contour of said article (6).

2. The method of forming a fiber reinforced article according to claim 1, wherein the molding step comprises radial compression molding.

3. The method of forming a fiber reinforced article according to claim 2, wherein the preform (1) is heated to above glass transition temperature (Tg) or melting temperature (Tm) of matrix polymer inside the radial molding apparatus before or during the molding step or before loading the preform (1) inside the radial molding apparatus, and cooled down during or after the molding step.

4. The method of forming a fiber reinforced article according to claim 1, wherein the molding step comprises injection molding, and where the obtained article (6) is a fiber insert, which is used as an insert in an overmolding process.

5. The method of forming a fiber reinforced article according to any preceding claims 1-4, wherein a distal end of the piston (4) comprises a mold with an inverse design which is reproduced at an end portion (6a) of the obtained fiber reinforced article (6).

6. The method of forming a fiber reinforced article according to any preceding claims 1-5, wherein the piston (5) is arranged to attach an insert or a metal or ceramic tip to the preform (1) during the compression.

7. The method of forming a fiber reinforced article according to any preceding claims 1-6, wherein the piston (4, 5) further comprises a sealing feature (7) at an interface of the mold cavity (3) arranged to seal the mold cavity (3) at the compressed position and/or during the molding step.

8. The method of forming a fiber reinforced article according to any preceding claims 1-7, wherein the initial volume of the preform (1) is smaller than volume of the mold cavity (3) in the initial position, and transverse dimension of the preform (1) in the initial position is larger than the transverse dimension of the obtained article (6) in the compressed position.

9. The method of forming a fiber reinforced article according to any preceding claims 1-8, wherein during the compression step, the die segments (2) move an identical linear or curved displacement length along the adjacent die segment (2), which movements are synchronized by at least one actuator, and wherein the die segments (2) are in direct contact with each other.

10. The method of forming a fiber reinforced article according to any preceding claims 1-9, wherein the fiber orientation of preform (1) can be tailored beforehand, wherein the preform (1) comprises a fiber insert (11) having uniaxial fiber orientation and plurality surrounding fiber layers (12) having a helix orientation with any desired fiber angle.

11. The method of forming a fiber reinforced article according to any preceding claims 1-10, wherein the preform (1) comprises an intermingled or interlocked layer structure.

12. The method of forming a fiber reinforced article according to any preceding claims 1-11, wherein the fiber reinforced article is a medical device.

13. A radial compression molding apparatus for forming a fiber reinforced article comprising thermoplastic polymer matrix and reinforcing fibers, wherein the apparatus comprises at least three adjacent die segments (2) next to each other forming a mold cavity (3) for a preform (1), wherein during a compression the die segments (2) move an identical linear or curved displacement length along the adjacent die segment (2), the die segments are arranged to be in direct contact with each other during an initial position and movement and a compressed position, which movements are synchronized by at least one actuator, and wherein the apparatus further comprises at least one piston (4, 5) arranged at an end of the mold cavity (3) and before or after the compression is arranged to slide towards the preform (1) inside the mold cavity (3) along a common longitudinal axis (A) of the preform (1).

14. The radial compression molding apparatus according to claim 13, wherein the distal end of the piston (4) comprises a mold with an inverse design.

15. The radial compression molding apparatus according to claim 13 or 14, wherein the piston (5) further comprises a metal or ceramic tip to be attached to an end of the preform (1) during the compression.

16. The radial compression molding apparatus according to any preceding claims 13-15, wherein the piston (4, 5) further comprises a sealing feature (7) at an interface of the mold cavity (3) arranged to seal the mold cavity (3) at a compressed position.

17. The radial compression molding apparatus according to claim 16, wherein the sealing feature (7) is a geometrical feature of the piston (4, 5), which is integrated or seamlessly joined to the piston (4, 5).

## Patentansprüche

1. Verfahren zur Herstellung eines faserverstärkten Artikels, das die folgenden Schritte umfasst:
101) Bereitstellen eines Verbundvorformlings (1), der eine thermoplastische Polymermatrix und Verstärkungsfasern umfasst, wobei der Vorformling (1) ein Anfangsvolumen und eine Anfangsfaserorientierung aufweist,
102) Einlegen des Vorformlings in eine Radialformvorrichtung, die mindestens drei benachbarte Matrizensegmente (2) nebeneinander umfasst, die einen Formhohlraum (3) in einer Anfangsposition mit einem Anfangsvolumen bilden,
103) Formen des Vorformlings (1) durch Bewegen der Matrizensegmente (2), die während der Anfangsposition und Bewegung und einer komprimierten Position in direktem Kontakt miteinander stehen und die senkrecht zu einer gemeinsamen Längsachse (A) des Vorformlings (1) stehen, wobei das Anfangsvolumen des Formhohlraums (3) abnimmt und die Matrizensegmente (2) den Vorformling (1) zu einer Form pressen, die durch den Formhohlraum (3) in der komprimierten Position definiert ist und mit einem Endvolumen, das kleiner oder gleich dem Anfangsvolumen des Vorformlings (1) ist,
wobei vor oder nach dem Schritt des Formens mindestens ein Kolben (4, 5) in Richtung des Vorformlings (1) innerhalb des Formhohlraums (3) entlang der gemeinsamen Längsachse (A) des Vorformlings (1) geschoben wird, um ferner das Pressen des Vorformlings (1) durch Abdichten des Formhohlraums (3) von mindestens einem Ende zu erleichtern,
104) Öffnen des Formhohlraums (3), und
105) Entfernen des erhaltenen faserverstärkten Artikels (6), der eine angepasste Faserorientierung umfasst, aus dem Formhohlraum (3), wobei die durchgehenden Verstärkungsfasern einer Oberflächenkontur des Artikels (6) folgen.

2. Verfahren zur Herstellung eines faserverstärkten Artikels nach Anspruch 1, wobei der Schritt des Formens das radiale Formpressen umfasst.

3. Verfahren zur Herstellung eines faserverstärkten Artikels nach Anspruch 2, wobei der Vorformling (1) vor oder während des Schritt des Formens oder vor dem Einlegen des Vorformlings (1) in die Radialformvorrichtung im Innern der Radialformvorrichtung auf eine Glasübergangstemperatur (Tg) oder Schmelztemperatur (Tm) von Matrixpolymer erwärmt und während oder nach dem Schritt des Formens abgekühlt wird.

4. Verfahren zur Herstellung eines faserverstärkten Artikels nach Anspruch 1, wobei der Schritt des Formens das Spritzgießen umfasst und wo der erhaltene Artikel (6) ein Fasereinleger ist, der als Einleger in einem Umspritzprozess verwendet wird.

5. Verfahren zur Herstellung eines faserverstärkten Artikels nach einem der vorstehenden Ansprüche 1 bis 4, wobei ein distales Ende des Kolbens (4) eine Form mit einer inversen Gestaltung umfasst, die an einem Endabschnitt (6a) des erhaltenen faserverstärkten Artikels (6) reproduziert wird.

6. Verfahren zur Herstellung eines faserverstärkten Artikels nach einem der vorstehenden Ansprüche 1 bis 5, wobei der Kolben (5) dafür ausgelegt ist, während der Kompression einen Einleger oder eine Metall- oder Keramikspitze an dem Vorformling (1) anzubringen.

7. Verfahren zur Herstellung eines faserverstärkten Artikels nach einem der vorstehenden Ansprüche 1 bis 6, wobei der Kolben (4, 5) ferner ein Dichtelement (7) an einer Grenzfläche des Formhohlraums (3) umfasst, das dafür ausgelegt ist, den Formhohlraum (3) in der komprimierten Position und/oder während des Schritts des Formens abzudichten.

8. Verfahren zur Herstellung eines faserverstärkten Artikels nach einem der vorstehenden Ansprüche 1 bis 7, wobei das Anfangsvolumen des Vorformlings (1) kleiner als das Volumen des Formhohlraums (3) in der Anfangsposition ist, und die Querabmessung des Vorformlings (1) in der Anfangsposition größer als die Querabmessung des erhaltenen Artikels (6) in der komprimierten Position ist.

9. Verfahren zur Herstellung eines faserverstärkten Artikels nach einem der vorstehenden Ansprüche 1 bis 8, wobei sich während des Schritt des Pressens die Matrizensegmente (2) um eine identische lineare oder gekrümmte Verschiebungslänge entlang des benachbarten Matrizensegments (2) bewegen, wobei die Bewegungen durch mindestens ein Stellglied synchronisiert werden, und wobei die Matrizensegmente (2) in direktem Kontakt miteinander stehen.

10. Verfahren zur Herstellung eines faserverstärkten Artikels nach einem der vorstehenden Ansprüche 1 bis 9, wobei die Faserorientierung des Vorformlings (1) im Voraus angepasst werden kann, wobei der Vorformling (1) einen Fasereinleger (11) mit uniaxialer Faserorientierung und einer Vielzahl von umgebenden Faserschichten (12) mit einer Helixorientierung mit einem beliebigen gewünschten Faserwinkel umfasst.

11. Verfahren zur Herstellung eines faserverstärkten Artikels nach einem der vorstehenden Ansprüche 1 bis 10, wobei der Vorformling (1) eine ineinandergreifende oder verschränkte Schichtstruktur aufweist.

12. Verfahren zur Herstellung eines faserverstärkten Artikels nach einem der vorstehenden Ansprüche 1 bis 11, wobei der faserverstärkte Artikel eine medizinische Vorrichtung ist.

13. Radialformpressvorrichtung zur Herstellung eines faserverstärkten Artikels, der eine thermoplastische Polymermatrix und Verstärkungsfasern umfasst,
wobei die Vorrichtung mindestens drei benachbarte Matrizensegmente (2) nebeneinander umfasst, die einen Formhohlraum (3) für einen Vorformling (1) bilden,
wobei sich die Matrizensegmente (2) während einer Pressung um eine identische lineare oder gekrümmte Verschiebungslänge entlang des benachbarten Matrizensegments (2) bewegen, wobei die Matrizensegmente dafür ausgelegt sind, während einer Anfangsposition und der Bewegung und einer komprimierten Position in direktem Kontakt miteinander zu stehen, wobei die Bewegungen durch mindestens ein Stellglied synchronisiert werden, und wobei die Vorrichtung ferner mindestens einen Kolben (4, 5) umfasst, der an einem Ende des Formhohlraums (3) angeordnet ist und dafür ausgelegt ist, vor oder nach der Pressung in Richtung des Vorformlings (1) innerhalb des Formhohlraums (3) entlang einer gemeinsamen Längsachse (A) des Vorformlings (1) zu gleiten.

14. Radialformpressvorrichtung nach Anspruch 13, wobei das distale Ende des Kolbens (4) eine Form mit einer inversen Gestaltung umfasst.

15. Radialformpressvorrichtung nach Anspruch 13 oder 14, wobei der Kolben (5) ferner eine Metall- oder Keramikspitze umfasst, die während der Pressung an einem Ende des Vorformlings (1) anzubringen ist.

16. Radialformpressvorrichtung nach einem der vorstehenden Ansprüche 13 bis 15, wobei der Kolben (4, 5) ferner ein Dichtelement (7) an einer Grenzfläche des Formhohlraums (3) umfasst, das dafür ausgelegt ist, den Formhohlraum (3) in einer komprimierten Position abzudichten.

17. Radialformpressvorrichtung nach Anspruch 16, wobei das Dichtelement (7) ein geometrisches Merkmal des Kolbens (4, 5) ist, das in den Kolben (4, 5) integriert oder nahtlos mit diesem verbunden ist.

## Revendications

1. Procédé de formation d'un article renforcé par des fibres, comprenant les étapes suivantes :
101) obtention d'une ébauche composite (1) comprenant une matrice de polymère thermoplastique et des fibres de renforcement, laquelle ébauche (1) comprend un volume initial et une orientation de fibres initiale,
102) chargement de l'ébauche à l'intérieur d'un appareil de moulage radial comprenant au moins trois segments de filière adjacents (2) proches les uns des autres en formant une cavité de moule (3) dans une position initiale ayant un volume initial,
103) moulage de l'ébauche (1) par déplacement des segments de filière (2), qui sont en contact direct les uns avec les autres durant la position initiale et le mouvement et une position compressée, et qui sont perpendiculaires à un axe longitudinal commun (A) de l'ébauche (1), dans lequel le volume initial de la cavité de moule (3) diminue et les segments de filière (2) compriment l'ébauche (1) sous une forme définie par la cavité de moule (3) dans la position compressée ayant un volume final, qui est inférieur ou égal au volume initial de l'ébauche (1),
dans lequel, avant ou après l'étape de moulage, au moins un piston (4, 5) est amené à coulisser en direction de l'ébauche (1) à l'intérieur de la cavité de moule (3) le long de l'axe longitudinal commun (A) de l'ébauche (1) pour faciliter davantage la compression de l'ébauche (1) en scellant la cavité de moule (3) depuis au moins une extrémité,
104) ouverture de la cavité de moule (3), et
105) retrait de l'article renforcé par des fibres obtenu (6), qui comprend une orientation de fibres personnalisée, hors de la cavité de moule (3), dans lequel les fibres de renforcement continues suivent un contour de surface dudit article (6).

2. Procédé de formation d'un article renforcé par des fibres selon la revendication 1, dans lequel l'étape de moulage comprend un moulage par compression radiale.

3. Procédé de formation d'un article renforcé par des fibres selon la revendication 2, dans lequel l'ébauche (1) est chauffée au-delà de la température de transition vitreuse (Tg) ou du point de fusion (Tm) du polymère de matrice à l'intérieur de l'appareil de moulage radial avant ou durant l'étape de moulage ou avant le chargement de l'ébauche (1) à l'intérieur de l'appareil de moulage radial, et refroidie durant ou après l'étape de moulage.

4. Procédé de formation d'un article renforcé par des fibres selon la revendication 1, dans lequel l'étape de moulage comprend un moulage par injection, et où l'article obtenu (6) est un insert de fibres, qui est utilisé en tant qu'insert dans un procédé de surmoulage.

5. Procédé de formation d'un article renforcé par des fibres selon l'une quelconque des revendications 1 à 4, dans lequel une extrémité distale du piston (4) comprend un moule avec un motif inverse qui est reproduit au niveau d'une portion d'extrémité (6a) de l'article renforcé par des fibres obtenu (6) .

6. Procédé de formation d'un article renforcé par des fibres selon l'une quelconque des revendications 1 à 5, dans lequel le piston (5) est disposé de manière à attacher un insert ou une pointe métallique ou céramique à l'ébauche (1) durant la compression.

7. Procédé de formation d'un article renforcé par des fibres selon l'une quelconque des revendications 1 à 6, dans lequel le piston (4, 5) comprend en outre un élément d'étanchéité (7) au niveau d'une interface de la cavité de moule (3), disposé de manière à sceller la cavité de moule (3) à la position compressée et/ou durant l'étape de moulage.

8. Procédé de formation d'un article renforcé par des fibres selon l'une quelconque des revendications 1 à 7, dans lequel le volume initial de l'ébauche (1) est inférieur au volume de la cavité de moule (3) dans la position initiale, et la dimension transversale de l'ébauche (1) dans la position initiale est supérieure à la dimension transversale de l'article obtenu (6) dans la position compressée.

9. Procédé de formation d'un article renforcé par des fibres selon l'une quelconque des revendications 1 à 8, dans lequel, durant l'étape de compression, les segments de filière (2) se déplacent sur une longueur de déplacement linéaire ou incurvée identique le long du segment de filière adjacent (2), lesquels déplacements sont synchronisés par au moins un actionneur, et dans lequel les segments de filière (2) sont en contact direct les uns avec les autres.

10. Procédé de formation d'un article renforcé par des fibres selon l'une quelconque des revendications 1 à 9, dans lequel l'orientation des fibres de l'ébauche (1) peut être personnalisée à l'avance, dans lequel l'ébauche (1) comprend un insert de fibres (11) ayant une orientation de fibres uniaxiale et une pluralité de couches de fibres environnantes (12) ayant une orientation en hélice selon n'importe quel angle souhaité des fibres.

11. Procédé de formation d'un article renforcé par des fibres selon l'une quelconque des revendications 1 à 10, dans lequel l'ébauche (1) comprend une structure de couches entremêlées ou imbriquées.

12. Procédé de formation d'un article renforcé par des fibres selon l'une quelconque des revendications 1 à 11, dans lequel l'article renforcé par des fibres est un dispositif médical.

13. Appareil de moulage par compression radiale pour former un article renforcé par des fibres comprenant une matrice de polymère thermoplastique et des fibres de renforcement, lequel appareil comprend au moins trois segments de filière adjacents (2) proches les uns des autres en formant une cavité de moule (3) pour une ébauche (1), dans lequel, durant une compression, les segments de filière (2) se déplacent sur une longueur de déplacement linéaire ou incurvée identique le long du segment de filière adjacent (2), les segments de filière sont disposés de manière à être en contact direct les uns avec les autres durant une position initiale et le mouvement et une position compressée, lesquels déplacements sont synchronisés par au moins un actionneur, et lequel appareil comprend en outre au moins un piston (4, 5) disposé au niveau d'une extrémité de la cavité de moule (3) et, avant ou après la compression, est disposé de manière à coulisser en direction de l'ébauche (1) à l'intérieur de la cavité de moule (3) le long d'un axe longitudinal commun (A) de l'ébauche (1).

14. Appareil de moulage par compression radiale selon la revendication 13, dans lequel l'extrémité distale du piston (4) comprend un moule avec un motif inverse.

15. Appareil de moulage par compression radiale selon la revendication 13 ou 14, dans lequel le piston (5) comprend en outre une pointe métallique ou céramique destinée à être attachée à une extrémité de l'ébauche (1) durant la compression.

16. Appareil de moulage par compression radiale selon l'une quelconque des revendications 13 à 15, dans lequel le piston (4, 5) comprend en outre un élément d'étanchéité (7) au niveau d'une interface de la cavité de moule (3), disposé de manière à sceller la cavité de moule (3) à une position compressée.

17. Appareil de moulage par compression radiale selon la revendication 16, dans lequel l'élément d'étanchéité (7) est un élément géométrique du piston (4, 5), qui est intégré ou joint sans soudure au piston (4, 5).
